# EUROPEAN PATENT SPECIFICATION

(11) **EP 0 526 862 B1**
(45) Date of publication and mention of the grant of the patent: **14.02.1996**
(21) Application number: 92113187.6
(22) Date of filing: 03.08.1992
(51) Int. Cl.: A61K 9/20, A61K 9/16, A61K 9/50

(54) **Solid pharmaceutical compositions for oral administration with prolonged gastric residence**
Feste Arzneizubereitungen zur oralen Verabreichung mit verlängerter Magenverweilzeit
Compositions pharmaceutiques solides pour l'administration orale à séjour gastrique prolongé

(30) Priority: 06.08.1991 IT MI912212
(43) Date of publication of application: 10.02.1993
(73) Proprietor: VECTORPHARMA INTERNATIONAL S.p.A., I-34100 Trieste (IT)
(72) Inventor: Esposito, Pierandrea, 34145 Trieste (IT); Carli, Fabio, I-34100 Trieste (IT)
(74) Representative: Gervasi, Gemma, Dr.

(56) References cited:
- EP-A- 0 173 210
- EP-A- 0 265 061

## Description

### PRIOR ART

Conventional pharmaceutical compositions for oral administration consist of one or more units, each of which contains a given quantity of active ingredient - mixed in the carrier -, whose formulation depends on the desired release.

Generally speaking, larger-sized pharmaceutical preparations (4-5 mm tablets and larger) are administered by single units, whilst smaller-sized pharmaceutical preparations (1-2 mm pellets, <1 mm granules, <200 µm microgranules) are administered as multiparticulate systems, usually enclosed in capsules, or suspended in water at the time of use.

The development of pharmaceutical compositions for oral administration has significantly progressed thanks to the production of controlled-release formulations.

Nevertheless, despite the possibility of adjusting the release of active ingredients, the attainment of an optimalized bioavailability of pharmaceuticals through absorption by the gastrointestinal tract is strongly dependent on the time of transit of pharmaceuticals in that very tract.

In particular, the time of residence in the stomach is a critical factor in the attainment of satisfactory hematic levels of drugs because of its being extremely subject to variation. Said variability is closely connected with the physiological conditions of the stomach (fed or fasted) and with the size of solid pharmaceutical formulations. For instance, if the release of a drug - aimed at a given absorption window - is comparatively slow, there is a possibility for that drug to pass beyond the optimal segment before the occurrence of the planned release. In this case, bioavailability will be lower than expected.

Gastric residence of pharmaceuticals plays a key role whenever the active ingredient to be administered falls within the classes of drugs meant for topical treatment (e.g. anti-ulcer drugs), or among drugs with reduced absorption window, chiefly located in the first section of the small intestine.

The possibility of securing a controlled increase in the gastric residence time and in the time of transit through the intestine was studied with pharmaceutical compositions based on different principles.

A composition based on a traditional principle is disclosed in patent PCT/US90/01933 granted on 10th April, 1990.

The invention refers to sustained release pharmaceuticals for oral administration including cross-linked polymers swelling when in contact with gastric fluids. These pharmaceuticals are capable of maintaining the physical integrity of the system during active ingredient release and dissolve quickly at a later stage. Swollen polymers reach such a size as to allow a prolonged residence in the stomach.

A principle widely applied in these last years is bioadhesion. Several patents were granted in connection with the invention of pharmaceutical compositions containing bioadhesives capable of being applied to the skin, or to the buccal or nasal mucosa simply by physician's or patient's light pressure on the affected surface.

US Pat. 4,226,848 and US Pat. 4,250,163, respectively granted on 10th February, 1980 and 7th October, 1981, deal with compositions for buccal or nasal application and consist of a matrix with various percentages of cellulose esters and acrylic acid homopolymers or copolymers. The active ingredient is dispersed in the matrix.

US Pat. 4,292,299, granted on 29th September, 1981, refers to a pharmaceutical composition with a sustained release of the active ingredient to be applied to the buccal or nasal mucosa, or to the skin, consisting of a layer of bioadhesive polymers swelling when in contact with biological fluids plus a non-adhesive layer of water-soluble ingredients. Active ingredients can be embodied both in the bioadhesive and water-soluble layer.

European Pat. EP 0159604, 30th October, 1985, refers to a sustained release pharmaceutical applicable to the oral cavity and consisting of different mixtures of two polymers. Said polymers are: (1) macromolecules selected among polyvinylpyrrolidone, polyvinyl alcohol, polyethylene glycol, alginic acid and respective salts and maleic anhydride and vinyl methyl ether copolymers, and (2) polymers belonging to the group of polyacrylic acids or respective salt. The bioadhesiveness of such systems was assessed by the use of mouse peritoneal membrane as biological material. Such a formulation can adhere to the oral cavity mucosa and ensure prolonged plasmatic levels of the embodied active ingredients.

US Pat. 4,572,832, granted on 25th February, 1986, refers to a soft buccal release formulation. The formulation consists of an active ingredient, a water-soluble protein, a polyhydric alcohol, a fat acid ester or a carboxyvinyl polymer. According to the inventors, the combination of such ingredients favours, among other properties, good retention by the oral cavity.

US Pat. 4,940,587, granted on 10th July, 1990, refers to a formulation meant for the oral or nasal mucosa, said formulation being coated by bioadhesive cellulose derivative.

Bioadhesive pharmaceutical compositions meant for gastrointestinal administration, at least as a possible alternative among several ways, are also described.

US Pat. 4,615,697, granted on 7th October, 1986, concerns a composition comprising a pharmacological component plus a bioadhesive. The bioadhesive is a cross-linked polymer containing carboxyl functions swelling when in contact with water, although water-insoluble. The composition can be in the form of particles sized below 149 µm (100 mesh, US Standard).

US Pat. 4,915,948, granted on 10th April, 1990, deals with a controlled-release improved-bioadhesion composition. This composition is produced in tablets containing effective amounts of bioadhesive water-soluble polymers (xanthane or pectins), as well as low molecular weight polyalcohols possibly with more than 20% solubility. Polyalcohols are meant to favour mucoadhesion, hence adhesiveness is higher than secured by polyalcohol-free bioadhesive formulations.

Another field of major interest in these last years is the use of high density oral formulations. Literature reports several studies conducted on man or animals. In spite of results being sometimes inconsistent, a great number of studies demonstrate that the administration of particulate pharmaceuticals with sufficiently high density lengthens gastric residence time in respect of particulate pharmaceuticals with lower density.

Among such studies, let us quote the one by Bechgaard et al., J. Pharm. Pharmacol., 30 (1978), 690-92, concerning the transit of pharmaceuticals containing particulates with density of 1.0 g/cm3 and 1.6 g/cm3 through the gastrointestinal tract of ileostomy patients. The higher the density of particulates, the considerably higher the total transit time.

As J. Meyer et al. say in Gastroenterology, 89 (1985), 805-13, in addition to size, the density of solid formulations too has a high impact on the residence time of same in the stomach.

Investigated densities were 1.6 g/cm3 and 2.0 g/cm3.

Belgian patent B 865,451, granted on 17th July, 1978, deals with oral pharmaceuticals in the form of pellets sized in the ranges from 0.3 to 0.7 mm and from 1.2 to 1.7 mm. The densities obtained are as high as 3.4 g/cm3, but trials with ileostomy patients were made only by the use of pellets with density up to 1.6 g/cm3. Difference in density seems to affect difference in the time of pellet transit through the intestine and, according to the authors, contributes to disperse the particulate along the intestine.

EP 0173210A, granted on 19th June, 1985, proposes an invention concerning a multiparticulate formulation (pellets), studied to increase the overall gastrointestinal transit time. The composition contains high density particulates plus binders and coating agents capable of withstanding the stomach acid environment. The patent reports data about the improved bioavailability and the lengthened Tmax of model pharmaceuticals (nifedipine).

In the patent it is clearly stated that the size of pellets ranges from 0.1 to 1.8 mm, pellet density being included between 1.4 and 2.4 g/cm3, preferably between 1.5 and 1.8 g/cm3.

UK Pat. GB 2 196 252, granted on 9th September, 1987, defines a high density composition with increased gastric residence time. In the patent it is clearly stated that values of 2 g/cm3 at least are required by a formulation to be defined high density. It is also affirmed that the size of the particles forming said composition must be equal to or larger than 2.0 mm if an effective gastric retention has to be secured. On the other hand, among the quoted examples, there are some concerning formulations with smaller particles, down to 0.5 to 0.8 mm. It is, anyway, affirmed that formulations containing so small or smaller particles are liable to be expelled from the stomach along with the fluids present, regardless of density, hence with no advantage over conventional formulations.

All the described techniques, however, are not sufficient to provide a satisfactory solution of the problem of securing an as prolonged as required residence of pharmaceutical compositions in the stomach.

Actually, if on the one hand high density formulations can reside in the gastric cavity longer than traditional formulations, on the other they are subject to the propulsive action of the stomach, where they are free to move. The higher the density, the lower the possibility for moving particles to reach the pyloric region, although once in that area formulations are expelled, density having lost its effect. Such a behaviour can be inferred from the time of initiation, halftime, and completion time of stomach emptying, as shown in UK Pat. GB 2 196 252: after a lag-time phase, the gastric emptying time results to be of an exponential or diphasic type. The time interval from the beginning to the end of emptying results to be of 89 minutes under fed conditions and 51 minutes under fasted conditions.

On the other hand, when for gastric use, bioadhesive formulations afford the following drawbacks:
1) an intrinsic difficulty to cause bioadhesive phenomena in a dynamic system subject to outstanding propulsive forces (gastric walls);
2) the impossibility of imparting an although miminum initial pressure to the formulation, which can instead be done in the case of buccal, nasal, vaginal bioadhesive formulations.

For such reasons, the time of residence in the stomach may fail to lengthen with said compositions.

### SUMMARY

New solid pharmaceutical compositions for oral administration have been developed. Unexpectedly, they exhibit a considerably prolonged time of residence in the stomach.

Such compositions are constituted by
1) one or several high density inorganics;
2) one or several bioadhesives;
3) an active ingredient, either as is or mixed in a carrier;
4) excipients, binders, lubricants and other materials commonly used in pharmaceutical formulations.

### DETAILED DESCRIPTION OF THE INVENTION

The properties and advantages of the solid pharmaceutical compositions for oral use with prolonged residence in the stomach, which are the subject of the present invention, are going to be better described hereinunder.

Such compositions can be in the form of single dosage units (tablets) or of multiple unit formulations (pellets, granules, microgranules) and consist of
1) one or several high density inorganics;
2) one or several bioadhesives;
3) an active ingredient, either as is or mixed in a carrier;
4) excipients, binders, lubricants and other materials commonly used in pharmaceutical formulations.

The high density matters that can be used according to the present invention are inorganic substances with density or specific gravity values ranging from 2 to 4 g/cm3 and over. Also substances with density of 6-7 g/cm3 and over can be used. Said substances are used in the form of solid particulates with average diameter ranging from 0.1 to 2-2.5 mm, or of tablets with average diameter ranging from 3 to 15 mm. Such matters must be present in compositions in concentrations sufficient to secure composition density exceeding 1.5 g/cm3, preferably from 2.0 to 3 g/cm3. Compositions with density of 4.0 g/cm3 and over can be prepared, although this is currently unnecessary. The concentration of high density substances must be 20% at least by weight, preferably between 30% and 70%, and can even reach 80% by weight.

Among the high density substances that may be used, let us mention the following, whose specific gravity is indicated in brackets in g/cm3: magnesium trisilicate (3.2); magnesium oxide (3.6); aluminium oxide (4.0); bismuth carbonate (6.3); bismuth nitrate (4.9); zinc oxide (5.6); titanium dioxide (3.9-4.2); calcium ferrite (5.1); ferrous oxide (5.7); barium sulphate (4.5); ferric oxide (4.5); metallic iron (7.8). High density substances can also include samarium oxide and hydroxide, erbium oxide and hydroxide, aluminium hydroxide, calcium carbonate, calcium aluminium silicate, magnesium carbonate, calcium phosphate, monobasic and dibasic phosphate, calcium sulphate, calcium oxide, magnesium oxide, magnesium hydroxide.

Particularly preferred high density substances are barium sulphate, metallic iron powder, samarium oxide, erbium oxide, magnesium trisilicate, aluminium trisilicate, magnesium aluminium trisilicate, titanium dioxide.

The amount of high density substance is a function of the desired density of the composition. Generally speaking, high density substances are contained inside the pharmaceutical formulation, although they can be dispersed in the film coating the pharmaceutical formulation, if any. Titanium dioxide can be referred to, by way of example, as it can be suspended in film-grade polymeric solutions.

The bioadhesives to be used in the compositions of the invention are generally polymers endowed with bioadhesive capacity. They can be present as single or combined materials. Bioadhesives can be added as binders or as matrix. Bioadhesive polymers can be present in the pharmaceutical also as a film coating the solid core. The bioadhesive coat and the binder or matrix can be simultaneously present to prolong the bioadhesive action.

Whenever used as binder or matrix, bioadhesives - whether polymers or not - can be present in formulations in percentages ranging from 10% to 60%, preferably from 20% to 40% by weight. In case one of the two components (high density substances or bioadhesives) is present in high amounts, the other can be present in smaller amounts than the above mentioned ones.

For example, in compositions with high content of high density substance, amounts of 2.0% by weight of bioadhesives (in mixture with other excipients as matrix or binder) are sufficient for obtaining the extension effect of the gastric residence time.

On the other hand compositions with a low content of high density substance can contain a bioadhesive amount up to 75% by weight.

When bioadhesives are present as solid core film coat, their percentage by weight can even be 1% or reach 20%, although it is preferably included in the 7% to 15% range.

The bioadhesives used according to the invention belong to two main categories, namely (1) swelling and water-soluble linear polymers, characterized by physical cross-linking; (2) swelling but water-insoluble polymers consisting of linear macromolecules interconnected by cross-linking agents. The said polymers exhibit different chemo-physical and swelling kinetic properties. A great number of these polymers can be used as matrixes or systems for active ingredient controlled release.

Lectins should also be mentioned among the bioadhesives covered by the invention.

The compositions prepared under the invention can be further enteric-coated with polymer film, which allows the obtainment of formulations capable of performing their bioadhesive action in the intestine, after the dissolution of the enteric coat. Among enteric coats, let us mention the various cellulose derivatives, such as cellulose acetate, cellulose triacetate, cellulose acetate butyrate, cellulose acetate phthalate, cellulose acetate trimellitate, and others such as the acrylic derivatives known under the trademark of Eudragit L or Eudragit S and, more generally, all coating materials the solubility of which rises with the intestinal pH.

The bioadhesives that can be used under the invention are: tragacanth gum, guar gum, acacia gum, scleroglucan, schizophillane, xanthane, chitosan, maleic anhydride and methylvinylether copolymers, and adhesive silicone gel, such as Dow Corning's product referred to as X1203E2. Some of said products are known under registered trademarks, such as, for instance, CARBOPOL, GANTREZ, ACTIGUM, SEA CURE, SATIAXANE.

The bioadhesives contained in the compositions prepared according to the invention can be formulated as pure polymer or pure material, or prepared as combinations of different bioadhesive polymers or as combinations of bioadhesive and non-bioadhesive polymers, or as combinations of bioadhesive polymers and plasticizers, swelling retarders, hardeners, hydrophobic agents, colouring agents. More generally, polymers or bioadhesive materials can be contained in formulations together with all the materials or excipients required by any specific pharmaceutical. In particular, combinations of two or more bioadhesive polymers - selected on the basis of their chemo-physical properties - can exhibit a synergic action as far as adhesion to the mucosa or the mucin layer is concerned, when compared with single materials. Among these combinations, let us mention, by way of example, mixtures consisting of 60% scleroglucan-40% alginate; 60% Carbopol-40% Gantrez 169; 60% scleroglucan-40% Gantrez; 60% Carbopol-40% scleroglucan.

Bioadhesive polymers may be accompanied by all those materials that are capable of increasing polymer adhesion to mucin and/or mucose.

Among said materials, let us mention, by way of example, low molecular weight polyols, such as xylenol, mannitol, sorbitol, or polymeric materials such as ethylcellulose.

The active ingredient (pharmaceutical) of compositions is contained as is or mixed in carriers meant either to improve the ingredient bioavailability properties or control the ingredient release by the desired kinetics.

Besides according to conventional methods, active ingredients can be formulated also according to the techniques developed and patented by Vectorpharma, as per patent applications Nos. 22770 A/88 and 22336 A/88.

These techniques allow the activation of the therapeutical ingredient, on a carrier, in an amorphous state or in nanometric-sized crystals. Such an activation makes the ingredient more quickly available for release to biological fluids.

The compositions according to the invention can be prepared with several classes of active ingredients. Some particular classes of these - such as, for instance, ingredients characterized by erratic gastrointestinal absorption or a limited absorption window located in specific tracts of the intestine, ingredients with short halftime as well as ingredients exerting a topical action on gastrointestinal mucosa or its annexes - can better benefit from said compositions.

Among the active ingredients better suiting the invention, let us mention those exerting a cardiovascular action, the ones acting on the central nervous system, non-steroid anti-inflammatory agents, anti-ulcer agents, antibiotics and antiviral agents, autacoids such as prostaglandins, ingredients for anticancer treatment.

A particularly interesting class of topical treatment drugs is that of anti-ulcer products including H1 and H2 antagonists as well as protonic pump inhibitors. H1 antagonists include ethanolamine derivatives (e.g. diphenhydramine), ethylenediamine derivatives (tripelennamine), alkylamine derivatives (chlorpheniramine maleate), piperazine derivatives, phenothiazine derivatives (promethazine). H2 antagonists include cimetidine and ranitidine, while homeprasole should be in particular quoted among protonic pump inhibitors. Anti-ulcer products are also inclusive of prostaglandins, carbenoxolane and sucralphate. A comprehensive class of pharmaceuticals that can be conveniently used according to the invention is that of cardiovasculars. The said class includes diuretics (e.g. chlorothiazide), peripheral vasodilators (e.g. hydralazine), coronary vasodilators (isosorbide, nitroglycerin), beta-blockers (metopropol), anticholesteraemic agents (e.g. clofibrate).

In particular, stress should be laid on the major role played, in the said class, by antiarrhythmic drugs, e.g. verapamil, propranolol, phenytoin, procainamide, amiodarone, quinidine.

Antihypertensive drugs are also covered by the invention. For instance, renin-angiotensin system inhibitors, such as ACE-inhibitors, result to be particularly interesting (e.g. captopril, enalapril).

Calcium antagonists, such as nifedipine, verapamil, diltiazem, are among the best suited groups of formulations covered by the invention, just as beta-blockers, whether aspecific (propranolol, nadolol, timolol, pindolol) or cardioselective (metoprolol).

Pharmaceuticals acting on the respiratory system can be successfully formulated by the methods described in the invention. Among these, let us mention antiasthmatic drugs, β2-adrenergics, e.g. salbutamol (albuterol), terbutaline, carbuterol, broxaterol, aminophylline, theophylline.

A great number of other pharmaceuticals and pharmaceutical classes can be prepared under the invention, such as, for instance:
antiemetic and antinauseant drugs (cyclizine, cinnarazine, domperidone, alizapride);
anticancer drugs, whether for topical action on gastrointestinal tumours, or for systemic action, such as vincristine, steroid derivatives like medroxyprogesterone acetate and megestrol acetate, antibiotics, e.g. daunorubicin, actinomycin, adriamycin, epipodophyllotoxins, e.g. etoposide and teniposide, antimetabolites, e.g. 5-fluorouracil;
non-steroid anti-inflammatory drugs (NSAID), such as acetylsalicylic acid, indomethacin, acemethacin, sulindac, piroxicam, ibuprofen, naproxen, ketoprofen;
central-action drugs, such as benzodiazepines (e.g. temazepam, lorazepam, flunitrazepam), antiparkinson agents (L-dopa, amantidine), tricyclic antidepressants (protriptyline hydrochloride, trimipramine maleate) and MAO-inhibitors (e.g. isocarboxazid), psychostimulants, antiepileptics;
cerebral vasodilators such as nicergoline;
antimicrobial agents such as antibacterials, antiviral drugs, and fungicides such as penicillin derivatives (eg ampicillin, amoxycillin), aminoglycosides, cephalosporins (cefalzolam, ceftriaxone), macrolides (erythromycin), tetracyclines, antiviral drugs used in the treatment of herpes infections (acyclovir, gancyclovir), and the latest developed anti-AIDS agents (e.g. zidovudin or azidothymidine).

The absorption of essential substances or food supplements such as vitamins or vitamin complexes, like riboflavin, cyanocobalamin, or aminoacids, or inorganic substances like iron or calcium salts or salts of essential trace elements like selenium can also be improved by the invention.

Active ingredients are also contained in the formulations covered by the invention in quantities ranging from 1 to 25% by wt.

Finally, binders, lubricants, plasticizers, colouring agents, preservatives, flavouring agents, and, in general, all materials fit for the preparation of formulations by the pharmaceutical practice can be used in the compositions covered by the invention. In particular, ethylcellulose, sometimes combined with bioadhesive polymers, can be used. Excipients can be inclusive of disintegrators, meant to cause the formulation to break up in the stomach only when the time desired for the active ingredient to be released has elapsed. Disintegrators can also be used to secure good dispersion of multiparticulate formulations in the stomach.

Lubricants consist of magnesium or calcium stearate, or vegetable oils, polyethylene glycols, talc, sodium lauryl sulphate, polyoxyethylene monostearate.

Binders include cellulose derivatives, such as ethylcellulose, hydroxyethylcellulose, hydroxypropyl cellulose, hydroxypropyl methylcellulose, microcrystalline cellulose, acrylic derivatives such as those known under the trademark of Eudragit, as well as polyvinylpyrrolidone.

The excipients of any type used according to the invention are dosed as required by pharmaceutical techniques.

### Formulation Procedures

As said at the beginning, the compositions according to the invention can be prepared in single units (tablets) or as multiparticulate formulations (pellets, granules, microgranules, microparticulates), hence with size ranging from tenths microns to 15 millimiters depending on the selected formulation form.

Single units (tablets) can have diameter from 3 to 15 mm, preferably from 3 to 7 mm. Tablets can be prepared according to standard techniques.

High density ingredients can be dry mixed, by stirring, with active ingredients, with bioadhesives and, if required, with binders and excipients, in one or more mixing steps. Tablets can be obtained by direct compression of the mixture obtained or of the granules obtained from the mixture.

When other techniques are used, the bioadhesive polymer or polymers can be used also, or solely, as tablet film coat. The thickness of said film is variable, as well as its wt. percentage - which may range from 1% to 20% - so as to secure a bioadhesive gelling coat and a layer for the controlled diffusion of the active ingredient.

Coating techniques are as commonly adopted in the production of pharmaceuticals, e.g. pan or fluidized bed coating.

A particular kind of tablet is the matrix type, where the bioadhesive polymer represents 30% to 60% by wt. of the composition.

The active ingredient can be either as is or mixed with an activating carrier, such as for instance crospovidone powder. The carrier/active ingredient system can be granulated or coformulated with high density materials and the binder - whether bioadhesive or not. The system thus obtained can then be dispersed in a bioadhesive gelling matrix.

Another kind of tablet is the so-called "tablet-in-the-tablet". When producing such a formulation, a small tablet - whether containing the active ingredient or not, but containing the high density material - is prepared and then inserted between two bioadhesive polymer layers containing the active ingredient, unless this is present in the small tablet. Then a second compression is performed.

This makes it possible to obtain a formulation capable of maintaining the bioadhesive polymer surface properties as unaltered as possible.

The initial density of formulations in the form of tablets is usually 2.5 to 3.0 g/cm3, although it may be as high as 3.5 g/cm3.

After swelling of the bioadhesive layer or of gelling polymers, density keeps around 1.8 to 2.0 g/cm3.

Multiparticulate formulations are based on the preparation of 1 to 3 mm pellets, 0.3 to 1 mm granules, and <0.3 mm microgranules.

Multiparticulate formulations can be prepared starting from a mixture consisting of high density material, variable permeability water-insoluble binder polymer, active ingredient as is or with carrier, excipients. Bioadhesive polymers may be present or not. The mixture is compacted with a suitable binder solution. The compacted mass can be later homogenized and extruded through variously sized dies. The extrudate is cut into pellets or granules of the desired size. Pellets and granules can also be obtained via the traditional spheronizing and pelletizing processes applied in the pharmaceutical industry.

The above described mixture can also be prepared by granulation using, for instance, fluidized bed granulators or other types of granulators.

Granules are sieved to obtain particulates of the desired size, both at granule and microgranule levels.

Microgranules covered by the invention can also be prepared with high density powders suspended in a solution of water-soluble bioadhesive polymers. Such polymer solutions are usually very viscous, which favours the suspension of particles. Said solutions contain the active ingredient as is or mixed in an activating carrier. The suspensions are then allowed to drip in a solvent, such as for instance ethyl alcohol, isopropyl alcohol, methylene chloride, where suspension drops promptly solidify, owing to polymer precipitation, thus incorporating in the solid both active ingredients and high density materials. This method usually allows the obtainment of <0.2 mm microparticulate formulations.

Whenever formed by a core not containing bioadhesives, multiparticulate formulations prepared according to the invention are coated with a polymer or bioadhesive material film. At any rate, also the multiparticulate formulations formed by a core containing bioadhesives can be coated likewise. The coating film may vary in thickness and by wt. percentage, although the content normally ranges from 7% to 15% of total weight; anyway, depending on the type of active ingredient used, coating film from 1% to 20% by weight can be obtained. The coating of multiparticulate formulations with bioadhesive film can be secured by the various current methods, e.g. fluidized bed, or pan, or spray-coating.

The initial density of multiparticulate formulations is equal to that of tablets, just as the density reached after hydration and swelling of bioadhesive materials.

The final form of multiparticulate formulations prepared according to the invention can be that of hard or soft gel capsules or of powder suitable for suspension in an appropriate medium at the time of administration.

For clarification purposes, examples are provided hereinunder with regard to composition preparation procedures according to the invention as well as to the check of the in vitro and in vivo behaviour of some of the prepared compositions tested on animals.

### EXAMPLE 1. Preparation of compositions by single compression.

**a)** A mixture having the following unit composition was prepared, after sieving of the single components (400 µm): nifedipine with micronized PVP-Cl (cross-linked polyvinylpyrrolidone) carrier (240 mg on a whole, 1:5 ratio), barium sulphate (235 mg), Methocel A4C (155 mg), Aerosil 200 (5 mg). After mixing, xanthane (Satiaxane CX 91, 30 mg) and galactomannan (Cesagum LN1, 30 mg), magnesium stearate (5 mg), suitably sieved, were added. After mixing, the powder was pre-compressed and compressed in 19 x 8 mm moulds to the obtainment of 700 mg tablets with 2.6 g/cm3 density.
**b)** Tablets containing titanium dioxide as high density agent were prepared likewise. Composition: 240 mg nifedipine/PVP-Cl, 1:5 ratio; 50 mg Satiaxane CX 91, 155 mg Methocel A4C, 5 mg Aerosil, 150 mg titanium dioxide. The mixture was pre-compressed and dry granulated on a 1.2 mm net, then sieved to 0.85 mm. Granules were compressed in 19 x 8 mm moulds, under 1500 kg, for the obtainment of 600 mg tablets with 2.3 g/cm3 density.
**c)** A mixture of 100 g barium sulphate/Eudragit RSPM, 95:5 ratio, was granulated by the wet way with a 10% water-ethyl alcohol solution of Eudragit RS. Captopril (20 g) was wet granulated with a scleroglucan solution (Actigum CS 11). Once dried and sieved (<175 µm), the two granulated components were mixed together and then with a further amount of scleroglucan, so as to secure a final granules/scleroglucan ratio equal to 80:20 by weight. The powder was compressed into 50 mg captopril tablets, 6.5 mm diameter, 300 mg total weight, with 2.5 g/cm3 density.
**d)** A procedure as per point c) was followed to obtain tablets containing 30 g barium sulphate, 2 g Eudragit RS, polyacrylic acid (Carbopol 934P). The active ingredient (verapamil, 10 g) was granulated with Eudragit RS and RL. Carbopol accounts for 30% of the composition. 100 mg verapamil tablets were obtained, 9.5 mm diameter, 2.5 g/cm3 density.

### EXAMPLE 2. Preparation of compositions by double compression.

**a)** A mixture of barium sulphate corresponding to about 90 mg per tablet and Eudragit RS PM, 95:5 ratio, was granulated by the wet way. Once sieved, granules were mixed with a given quantity of salbutamol sulphate (4 mg per dose) granulated with a 1% scleroglucan solution. The mixture was compressed under 1000 kg/cm2 for the obtainment of 5 mm diameter and 100 mg weight tablets. The thus prepared tablets (high density core) were placed in a 6.5 mg punch, between two scleroglucan powder layers, 60 mg, and further compressed under 1000 kg/cm2 for the obtainment of a "tablet-in-the-tablet" (3.0 g/cm3 density).
**b)** A composition like that described under point a) was prepared, in which the active ingredient, salbutamol, is present in the core (2.0 mg) and physically dispersed in the outer polymer matrix (1.5 mg) consisting of xanthane (Satiaxane CX 91) (2.8 g/cm3 density).

### EXAMPLE 3. Preparation of multiparticulate formulations according to the prior art (comparison).

High density multiparticulate formulations not containing bioadhesive polymers or materials were prepared for comparison purposes.
**a)** A mixture of 100 g barium sulphate and Eudragit RS (15:85 ratio) was granulated with a 10% Eudragit RS water-ethyl alcohol solution. The moist mass was extruded through a die with 2 or 1 mm diameter and dried at 45 to 50°C. The 2 mm particles thus obtained were spheronized so as to give pellets with 2 mm diameter. Smaller particles (1 mm or below) were granulated and classified in various fractions (0.8 to 1 mm; 0.5 to 0.8 mm; <0.4 mm).
   The density of said formulation, measured by the volume displacement system, was equal to 3.0 g/cm3.
**b)** Multiparticulate formulations with 1.6 g/cm3 density were prepared as described under point a). The extruded mass, granulated with a water-ethyl alcohol solution of Eudragit RS, consisted of 10% barium sulphate, 70% calcium sulphate, 5% magnesium stearate, and 15% Eudragit RS PM.

### EXAMPLE 4. Preparation of matrix-based multiparticulate formulations.

**a)** A mixture of 65% barium sulphate, 5% Eudragit RS, 8% Eudragit RL, 12% Satiaxane CX 91, 10% piroxicam with beta-cyclodextrin carrier (1:2.5) was prepared. The mixture was granulated with a 10% Eudragit RS solution (65:35 ethyl alcohol-water ratio). The moist mass was extruded through a 2 mm diameter orifice and cut. After drying at 55°C, the particulate was spheronized into pellets with 2 mm diameter, with 2.6 to 2.8 g/cm3 density.
**b)** A formulation like that described under point a) was prepared consisting of a mixture of 70% barium sulphate, 4 % Eudragit RS, 15% Actigum CS 11, 1% magnesium stearate, 10% alizapride. The mixture was granulated with a 1% scleroglucan solution. The procedure was carried on as described in Example 4a) (2.7 g/cm3 density).

### EXAMPLE 5. Preparation of bioadhesive film-coated multiparticulate formulations. Pellets.

**a)** A mixture of 70% barium sulphate, 8% Eudragit RL, 2% Eudragit RS, 20% cimetidine was prepared. The mixture was granulated with a 10% Eudragit RS water-ethyl alcohol solution. The moist mass was extruded, dried, cut, and pelletized as described in Example 4. The 2 mm pellets obtained were subjected to a pan coating process. The coating solution consisted of scleroglucan (Actigum CS 11) and an ethyl cellulose latex (Ethocel, Aquacoat E30), in a solid mass ratio equal to 80:20. Pellets were pan sprayed with the solution so as to obtain a bioadhesive polymer film accounting for 8% of the overall pellet mass. Pellet density was 2.6 g/cm3.
**b)** A composition containing 20% etoposide with polyvinylpyrrolidone carrier (1:5 ratio) was prepared according to the procedure and with the materials described under point a). The pellets obtained were sprayed with a polymer solution containing Satiaxane CX 90 HV and Aquacoat E30, in a solid mass ratio equal to 85:15. The bioadhesive film accounted for 10% of the overall pellet mass. Pellet density was 2.4 g/cm3.

### EXAMPLE 6. Preparation of bioadhesive film-coated multiparticulate formulations. Granules.

**a)** A powder mixture of 70% barium sulphate, 10% Eudragit RL, 1% magnesium stearate, 19% etoposide with micronized PVP-Cl (cross-linked polyvinylpyrrolidone) carrier (1:5 ratio) was prepared. The mixture was granulated with a 10% Eudragit RS water-ethyl alcohol solution. The moist mass was extruded through 1 mm or 0.6 mm orifices, then it was dried and cut. Granulation yielded regular shaped granules, which were subdivided into size classes.
   0.8 to 1 mm granules were pan film-coated with Carbopol 934P and polyethylene glycol (PEG 600) dispersion, in a solid mass ratio equal to 80:20.
**b)** Granules obtained as above were coated with a scleroglucan (Actigum CS 11) solution containing ethyl cellulose (Ethocel E4M), in an 80:20 ratio, according to the procedure described under point a). The coating film accounts for 15% of granule weight.
   The initial density of granules was 2.5 g/cm3 and after swelling, it was 1.8 to 2.1 g/cm3.
**c)** A mixture like that described under point 6 a) was prepared. The mixture contained ranitidine with micronized PVP-Cl carrier (1:5 ratio), accounting for 10% of the dry mass weight. The mixture was granulated in a fluidized bed granulator to obtain 0.4 to 0.2 mm diameter granules.
   The granules thus obtained were spray-coated with a scleroglucan-ethylcellulose solution, in a 75:25 ratio. The film coat accounted for 10% of granule weight.
**d)** A mixture like that described under point 6 c) was prepared. The mixture contained barium sulphate, Actigum CS 11, Ethocel, the active ingredient being enalapril maleate with PVP-Cl carrier (1:5 or 1:10 ratio).
   The fluidized-bed granulating agent was a 0.5% Actigum CS 11 solution. The granules thus obtained were spray-coated with a 1% sodium carboxymethylcellulose solution. The coating film accounted for 5% of the granule weight.
**e)** Granules as described under point 6 d) were spray-coated with a Gantrez AN 169 solution.

### IN VIVO TESTS

The pharmaceutical compositions prepared according to the invention were assessed through tests on animals by gamma scintigraphy, a technique allowing the quantitative monitoring of the position and behaviour of one composition - or two simultaneously - in the gastrointestinal tract as well as in other biological regions.

The composition used for tests were labelled with radioactive isotopes (indium 111 and technetium 99m), so as to identify the source of radioactivity with the pharmaceutical form in any moment.

Times and characteristics of transit through the gastrointestinal tract were studied and compared with the behaviour of formulations according to the prior art (high density formulations).

Results were plotted on a diagram as fraction of radioactivity present in the stomach corresponding to a fraction of pharmaceutical composition, vs. time. Parameters such as gastric residence time (fraction of radioactivity in the stomach) and the area under the curve (AUC) showing the amount of composition "available" in the stomach were evaluated.

The compositions prepared according to the invention evidenced a clearcut lengthening of the gastric residence time when compared with bioadhesive-free high density formulations. The comparison was conducted on two compositions of different density, i.e. 1.6 and 3.0 g/cm3 respectively. A longer time of residence in the gastric environment was recorded in both of the stomach physiological conditions (fasted and fed), the latter being more significant. Such an increase ranged from a minimum of 30% to over 300% with respect to the comparative formulations. Variability can be imputed to the different effectiveness of various combinations of high density materials and bioadhesives as well as to the intrinsic properties of the latter.

Longer residence times were recorded with all the tested pharmaceuticals prepared according to the invention. Anyway, a greater effectiveness was found to be secured by granular multiparticulate formulations with size equal to or smaller than 2 mm, in particular by granules sized from 1 mm to 0.5 mm.

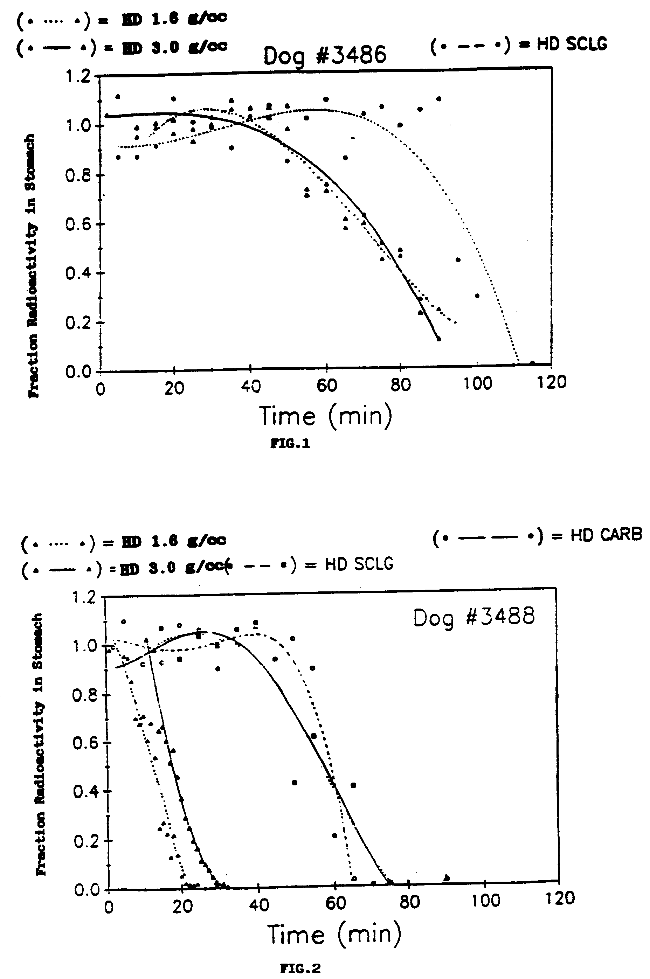

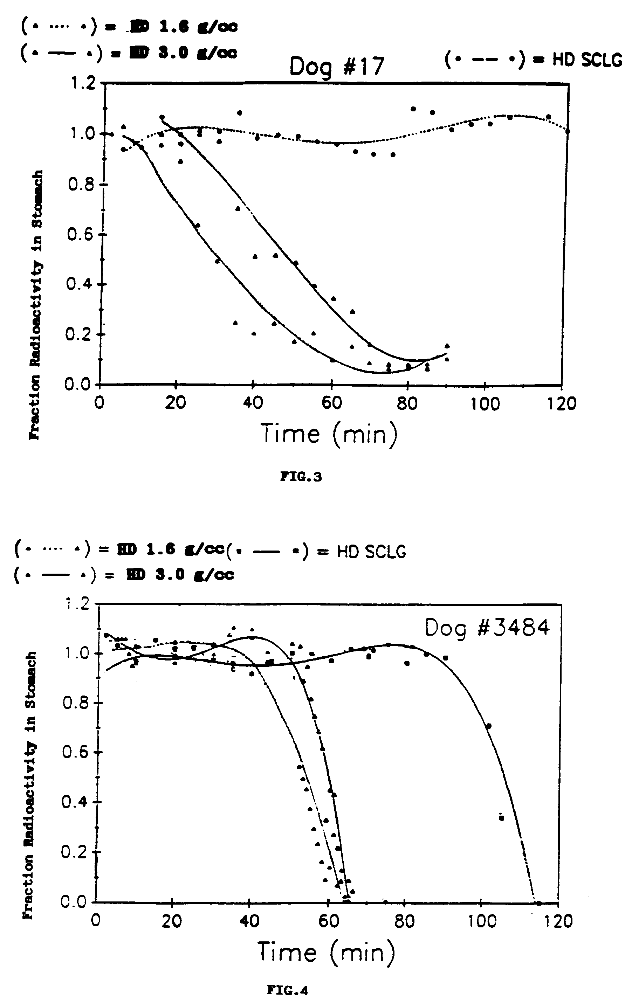

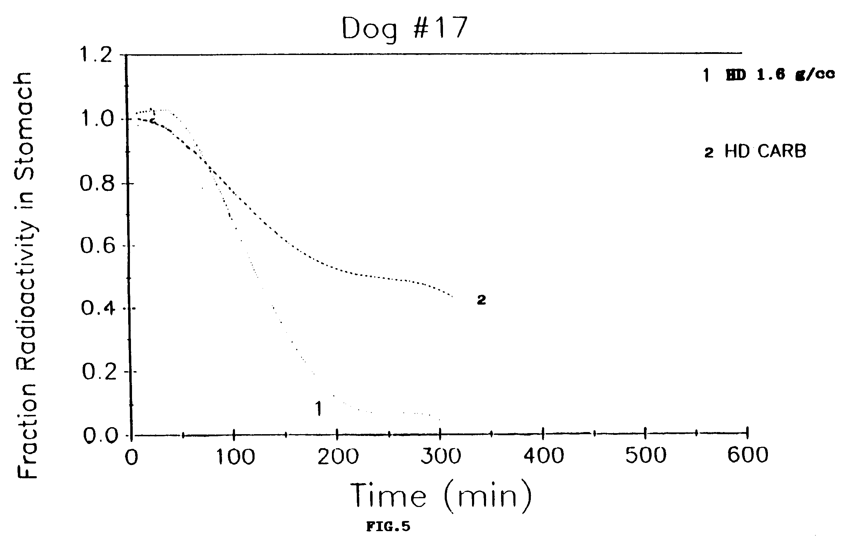

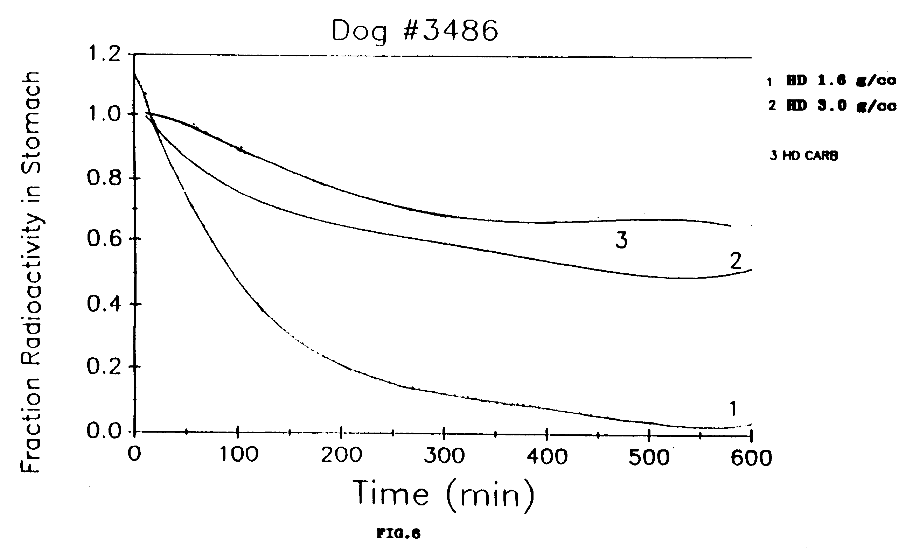

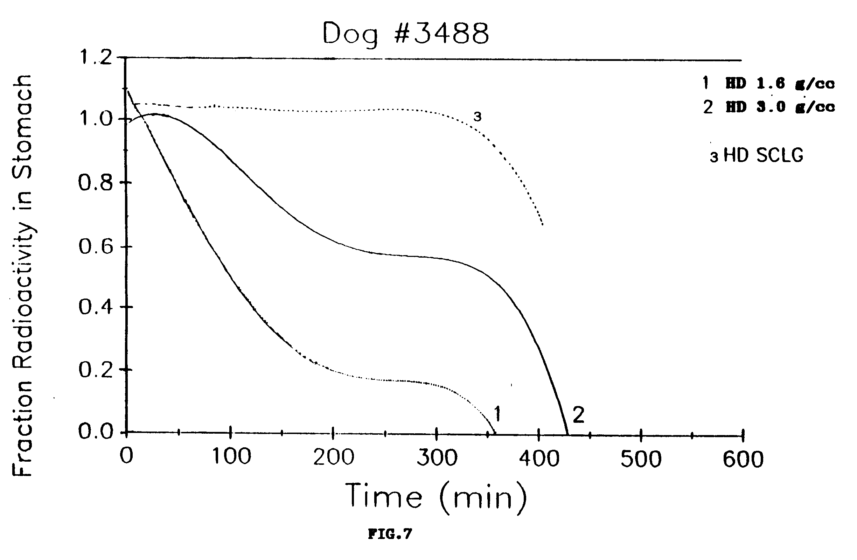

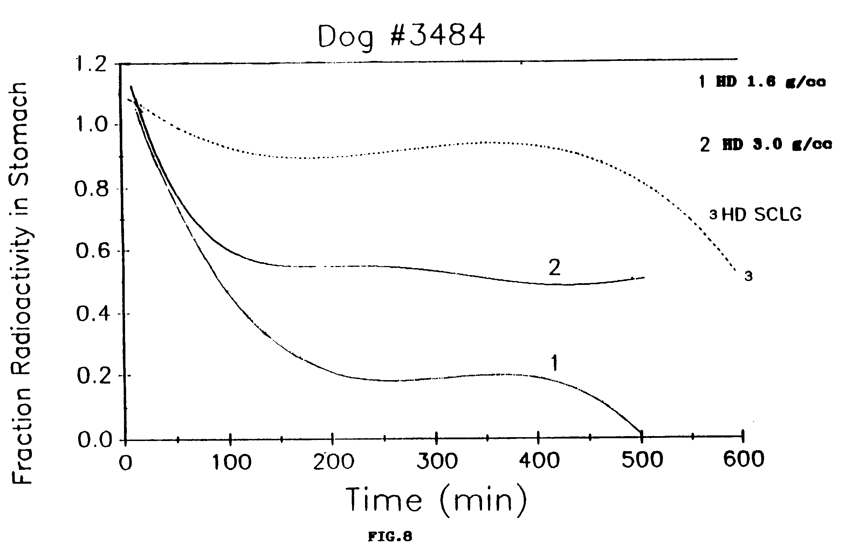

The following is reported by way of example:
1) Four fasted dogs' stomach emptying curves with compositions in the form of 0.8-1 mm film coated granules respectively containing scleroglucan and Carbopol as bioadhesive polymers (Figs. 1-4). The compositions according to the invention, prepared as respectively described in Example 6b) and Example 6a), were indicated as HDSCLG and HDCARB.
   The comparative compositions, prepared as described in Example 3 (a-c), were indicated as HD 1.6 g/cm3 and HD 3.0 g/cm3.
2) Four fed dogs' stomach emptying curves with 0.8-1 mm granules containing sclerogucan and Carbopol as bioadhesive polymers (Figs. 5-8). The symbols marking the various compositions have the same meaning as indicated under point 1).
3) Recapitulative table of test results (Table 1), where the AUC's calculated for granules (0.8-1 mm) according to the invention respectively containing Carbopol (HDCARB) and scleroglucan (HDSCLG) were compared with the AUC's of HD 3.0 g/cm3 and HD 1.6 g/cm3 comparative granules.

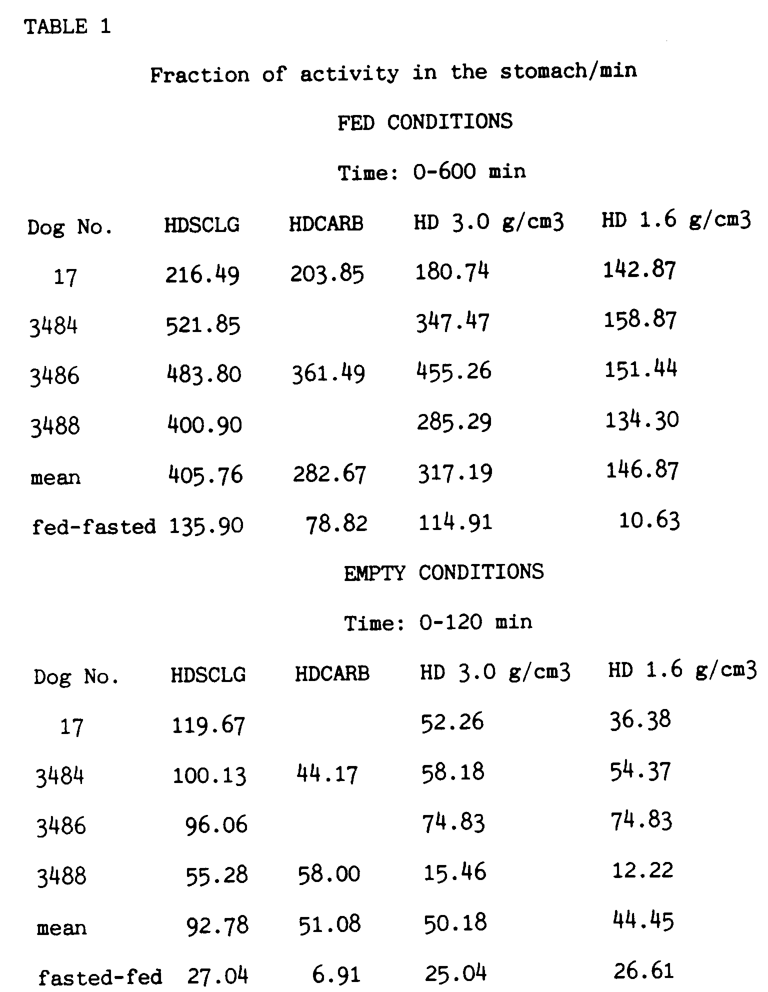

## Claims (Claims for the following Contracting State(s): AT, BE, CH, LI, DE, DK, FR, GB, IT, LU, MC, NL, PT, SE, IE)

1. Solid pharmaceutical compositions for oral administration, with prolonged gastric residence, comprising one or more high density inorganic substances, one or more bioadhesives, an active ingredient - either as is or mixed in a carrier -, as well as excipients, binders, lubricants and other materials commonly used in pharmaceutical formulations, characterized in that said bioadhesives are selected from the group consisting of tragacanth gum, guar gum, acacia gum, scleroglucan, schizophillane, xanthane, chitosan, maleic anhydride and methylvinylether copolymers, and adhesive silicone gel.

2. Pharmaceutical compositions as per claim 1, wherein said inorganic substances have density ranging from 2 to 7 g/cm3.

3. Pharmaceutical compositions as per claim 1, wherein said inorganic substances are used in the form of particulate solids with average size from 0.1 to 2.5 mm.

4. Pharmaceutical compositions as per claim 1, having density ranging from 1.5 to 4 g/cm3.

5. Pharmaceutical compositions as per claim 1, wherein said inorganic substances account from 20 to 80% by weight.

6. Pharmaceutical compositions as per claim 1, wherein said inorganic substances are selected from the group consisting of magnesium trisilicate, magnesium oxide, aluminium oxide, bismuth carbonate, bismuth nitrate, zinc oxide, titanium dioxide, calcium ferrite, ferrous oxide, barium sulphate, ferric oxide, metallic iron, samarium oxide and hydroxide, erbium oxide and hydroxide, aluminium hydroxide, calcium carbonate, calcium aluminium silicate, magnesium carbonate, calcium phosphate, monobasic and dibasic phosphate, calcium sulphate, calcium oxide, magnesium oxide, magnesium hydroxide.

7. Pharmaceutical compositions as per claim 1, wherein said inorganic substances are formulated inside the pharmaceutical form.

8. Pharmaceutical compositions as per claim 1, wherein said inorganic substances are dispersed in a film coating the pharmaceutical form.

9. Pharmaceutical compositions as per claim 1, wherein said bioadhesives are present as matrix or binder in percentages ranging from 2.0% to 75% by weight.

10. Pharmaceutical compositions as per claim 1, wherein said bioadhesives are present as external coating of the solid form in percentages ranging from 1% to 20% by weight.

11. Pharmaceutical compositions as per claim 1, wherein said active ingredient is selected from the group consisting of diphenhydramine, tripenellamine, chlorpheniramine maleate, promethazine, cimetidine, ranitidine, homeprasole, prostaglandine, carbenoxolane, sucralphate, chlorothiazide, hydralazine, isosorbide, nitroglycerin, metoprolol, clofibrate, verapamil, propranolol, phenytoin, procainamide, amiodarone, quinidine, captopril, enalapril, nifedipine, verapamil, diltiazem, nadolol, timolol, pindolol, salbutamol, terbutaline, carbuterol, broxaterol, aminophylline, theophylline, cyclizine, cinnarizine, domperidone, alizapride, vincristine, medroxyprogesterone acetate, megestrol acetate, daunorubicin, actinomycin, adriamycin, etoposide and teniposide, 5-fluorouracil, acetylsalicylic acid, indomethacin, acemetacin, sulindac, piroxicam, ibuprofen, naproxen, ketoprofen, temazepam, lorazepam, flunitrazepam, dopa, amantadine, protriptyline hydrochloride, trimipramine maleate, isocarboxazid, nicergoline, ampicillin, amoxycillin, aminoglycosides, cefazolam, ceftriaxone, erythromycin, tetracyclines, acyclovir, gancyclovir, zidovudine, riboflavin, cyanocobalamin, aminoacids, iron or calcium salts, salts of essential trace elements.

12. Pharmaceutical compositions as per claim 1, prepared in the form of tablets.

13. Pharmaceutical compositions as per claim 1, prepared in the form of multiparticulate formulations sized from <0.3 mm to 3 mm.

14. Pharmaceutical compositions as per claim 1, comprising a bioadhesive-free core coated with a bioadhesive film.

## Claims (Claims for the following Contracting State(s): ES, GR)

1. Process for the preparation of solid pharmaceutical compositions for oral administration, with prolonged gastric residence, comprising one or more high density inorganic substances, one or more bioadhesives, an active ingredient - either as is or mixed in a carrier -, as well as excipients, binders, lubricants and other materials commonly used in pharmaceutical formulations, wherein said high density ingredients are dry mixed, by stirring, with said active ingredients, bioadhesives, binders, excipients, lubricants and other materials and the mixture is converted to compositions in single units, tablets, or to multiparticulate formulations, pellets, granules, microganules and microparticulates.

2. Process as per claim 1, wherein said bioadhesives are selected from the group consisting of tragacanth gum, guar gum, acacia gum, scleroglucan, schizophillane, xanthane, chitosan, maleic anhydride and methylvinylether copolymers, and adhesive silicone gel.

3. Process as per claim 1, wherein said inorganic substances have density ranging from 2 to 7 g/cm3.

4. Process as per claim 1, wherein said inorganic substances are used in the form of particulate solids with average diameter from 0.1 to 2.5 mm.

5. Process as per claim 1, wherein said inorganic substances account from 20 to 80% by weight.

6. Process as per claim 1, wherein said inorganic substances are selected from the group consisiting of magnesium trisilicate, magnesium oxide, aluminium oxide, bismuth carbonate, bismuth nitrate, zinc oxide, titanium dioxide, calcium ferrite, ferrous oxide, barium sulphate, ferric oxide, metallic iron, samarium oxide and hydroxide, erbium oxide and hydroxide, aluminium hydroxide, calcium carbonate, calcium aluminium silicate, magnesium carbonate, calcium phosphate, monobasic and dibasic phosphate, calcium sulphate, calcium oxide, magnesium oxide, magnesium hydroxide.

7. Process as per claim 1, wherein said bioadhesives are present as matrix or binder in percentages ranging from 2.0% to 75% by weight.

8. Process as per claim 1, wherein said active ingredient is selected from the group consisting of diphenhydramine, tripenellamine, chlorpheniramine maleate, promethazine, cimetidine, ranitidine, homeprasole, prostaglandine, carbenoxolane, sucralphate, chlorothiazide, hydralazine, isosorbide, nitroglycerin, metoprolol, clofibrate, verapamil, propanolol, phenytoin, procainamide, amiodarone, quinidine, captopril, enalapril, nifedipine, verapamil, diltiazem, nadolol, timolol, pindolol, salbutamol, terbutaline, carbuterol, broxaterolo, aminophylline, theophylline, cyclizine, cinnarizine, domperidone, alizapride, vincristine, medroxyprogesterone acetate, megestrol acetate, daunorubicin, actinomycin, adriamycin, etoposide and teniposide, 5-fluorouracil, acetylsalicylic acid, indomethacin, acemetacina, sulindac, piroxicam, ibuprofen, naproxen, ketoprofen, temazepam, lorazepam, flunitrazepam, dopa, amantadine, protriptyline hydrochloride, trimipramine maleate, isocarboxazid, nicergoline, ampicillin, amoxycillin, aminoglycosides, cefazolam, ceftriaxone, erythromycin, tetracyclines, acyclovir, gancyclovir, zidovudine, riboflavin, cyanocobalamin, aminoacids, iron or calcium salts, salts of essential trace elements.

9. Process as per claim 1, wherein said compositions are prepared in the form of multiparticulate formulations sized from <0.3 mm to 3 mm.

## Patentansprüche (Patentansprüche für folgende(n) Vertragsstaat(en): AT, BE, CH, LI, DE, DK, FR, GB, IT, LU, MC, NL, PT, SE, IE)

1. Feste pharmazeutische Zusammensetzungen für die orale Verabreichung mit verlängerter Magenverweilzeit, umfassend eine oder mehrere anorganische Substanzen hoher Dichte, ein oder mehrere Bioadhäsive, einen aktiven Bestandteil - entweder so wie er ist oder in einem Träger vermischt - ebenso wie Exzipienten, Bindemittel, Gleitmittel und andere Materialien, die in pharmazeutischen Formulierungen allgemein verwendet werden,
dadurch **gekennzeichnet**, daß
die Bioadhäsive ausgewählt sind aus der Gruppe, bestehend aus Tragacanthgummi, Guar-Gummi, Akazia-Gummi, Skleroglucan, Schizophillan, Xanthan, Chitosan, Maleinsäureanhydrid und Methylvinylether-Copolymeren und adhäsivem Silikongel.

2. Pharmazeutische Zusammensetzungen nach Anspruch 1, worin die anorganischen Substanen eine Dichte im Bereich von 2 bis 7 g/cm³ haben.

3. Pharmazeutische Zusammensetzungen nach Anspruch 1, worin die anorganischen Substanzen in der Form von körnigen Feststoffen mit einer durchschnittlichen Größe von 0,1 bis 2,5 mm verwendet sind.

4. Pharmazeutische Zusammensetzungen nach Anspruch 1, mit einer Dichte im Bereich von 1,5 bis 4 g/cm³.

5. Pharmazeutische Zusammensetzungen nach Anspruch 1, worin die organischen Substanzen eine Menge von 20 bis 80 Gew.% ausmachen.

6. Pharmazeutische Zusammensetzungen nach Anspruch 1, worin die anorganischen Substanzen ausgewählt sind aus der Gruppe, bestehend aus Magnesiumtrisilikat, Magnesiumoxid, Aluminiumoxid, Wismutcarbonat, Wismutnitrat, Zinkoxid, Titandioxid, Calciumferrit, Ferrooxid, Bariumsulphat, Ferrioxid, metallisches Eisen, Samariumoxid und -hydroxid, Erbiumoxid und -hydroxid, Aluminiumhydroxid, Calciumcarbonat, Calciumaluminiumsilikat, Magnesiumcarbonat, Calciumphosphat, monobasischem und dibasischem Phosphat, Calciumsulphat, Calciumoxid, Magnesiumoxid, Magnesiumhydroxid.

7. Pharmazeutische Zusammensetzungen nach Anspruch 1, worin die anorganischen Substanzen innerhalb der pharmazeutischen Form formuliert sind.

8. Pharmazeutische Zusammensetzungen nach Anspruch 1, worin die anorganischen Substanzen in einem Film dispergiert sind, der die pharmazeutische Form beschichtet.

9. Pharmazeutische Zusammensetzungen nach Anspruch 1, worin die Bioadhäsive als Matrix oder Bindemittel in Prozentsätzen im Bereich von 2,0 bis 75 Gew.% vorhanden sind.

10. Pharmazeutische Zusammensetzungen nach Anspruch 1, worin die Bioadhäsive als externe Beschichtung der festen Form in Prozentsätzen im Bereich von 1 bis 20 Gew.% vorhanden sind.

11. Pharmazeutische Zusammensetzungen nach Anspruch 1, worin der aktive Bestandteil ausgewählt ist aus der Gruppe, bestehend aus Diphenhydramin, Tripenellamin, Chlorpheniraminmaleat, Promethazin, Cimetidin, Ranitidin, Homeprasol, Prostaglandin, Carbenoxolan, Sucralphat, Chlorthiazid, Hydralazin, Isosorbid, Nitroglycerin, Metoprolol, Chlofibrat, Verapamil, Propranolol, Phenytoin, Procainamid, Amiodaron, Quinidin, Captopril, Enalapril, Nifedipin, Verapamil, Diltiazem, Nadolol, Timolol, Pindolol, Salbutamol, Terbutalin, Carbuterol, Broxaterol, Aminophyllin, Theophyllin, Cyclizin, Cinnarizin, Domperidon, Alizaprid, Vincristin, Medroxyprogesteronacetat, Megestrolacetat, Daunorubicin, Actinomycin, Adriamycin, Etoposid und Teniposid, 5-Fluorouracil, Acetylsalicylsäure, Indomethacin, Acemetacin, Sulindac, Piroxicam, Ibuprofen, Naproxen, Ketoprofen, Temazepam, Lorazepam, Flunitrazepam, Dopa, Amantadin, Protriptylin-Hydrochlorid, Trimipraminmaleat, Isocarboxazid, Nicergolin, Ampicillin, Amoxycillin, Aminoglycosiden, Cefazolam, Ceftriaxon, Erythromycin, Tetracyclinen, Acyclovir, Gancyclovir, Zidovudin, Riboflavin, Cyanokobalamin, Aminosäuren, Eisen- oder Kalciumsalzen, Salzen von essentiellen Spurenelementen.

12. Pharmazeutische Zusammensetzungen nach Anspruch 1, hergestellt in der Form von Tabletten.

13. Pharmazeutische Zusammensetzungen nach Anspruch 1, hergestellt in der Form von multikörnigen Formulierungen mit einer Größe von < 0,3 mm bis 3 mm.

14. Pharmazeutische Zusammensetzungen nach Anspruch 1, umfassend einen bioadhäsivfreien Kern, der mit einem bioadhäsiven Film beschichtet ist.

## Patentansprüche (Patentansprüche für folgende(n) Vertragsstaat(en): ES, GR)

1. Verfahren zur Herstellung von festen pharmazeutischen Zusammensetzungen für die orale Verabreichung mit verlängerter Magenverweilzeit, umfassend eine oder mehrere anorganische Substanzen hoher Dichte, einen oder mehrere Bioadhäsive, einen aktiven Bestandteil - entweder so wie er ist oder in einem Träger vermischt - ebenso wie Exzipienten, Bindemittel, Gleitmittel und andere Materialien, die in pharmazeutischen Formulierungen allgemein verwendet werden, worin die Bestandteile hoher Dichte durch Rühren mit den aktiven Bestandteilen, Bioadhäsiven, Bindemittel, Exzipienten, Gleitmitteln und anderen Materialien trocken vermischt werden und die Mischung zu Zusammensetzungen in einzelnen Einheiten, Tabletten oder multikörnigen Formulierungen, Pellets, Körnchen, Mikrokörnchen und Mikroteilchen umgewandelt wird.

2. Verfahren nach Anspruch 1,
worin die Bioadhäsive ausgewählt sind aus der Gruppe, bestehend aus Tragacanthgummi, Guar-Gummi, Akazia-Gummi, Skleroglucan, Schizophillan, Xanthan, Chitosan, Maleinsäureanhydrid und Methylvinylethercopolymeren und adhäsivem Silikongel.

3. Verfahren nach Anspruch 1, worin die anorganischen Substanen eine Dichte im Bereich von 2 bis 7 g/cm³ haben.

4. Verfahren nach Anspruch 1,
worin die anorganischen Substanzen in der Form von körnigen Feststoffen mit einem durchschnittlichen Durchmesser von 0,1 bis 2,5 mm verwendet werden.

5. Verfahren nach Anspruch 1, worin die anorganischen Substanzen eine Menge von 20 bis 80 Gew.% ausmachen.

6. Verfahren nach Anspruch 1, worin die anorganischen Substanzen ausgewählt sind aus der Gruppe, bestehend aus Magnesiumtrisilikat, Magnesiumoxid, Aluminiumoxid, Wismutcarbonat, Wismutnitrat, Zinkoxid, Titandioxid, Calciumferrit, Ferrooxid, Bariumsulphat, Ferrioxid, metallischem Eisen, Samariumoxid und -hydroxid, Erbiumoxid und -hydroxid, Aluminiumhydroxid, Calciumcarbonat, Calciumaluminiumsilikat, Magnesiumcarbonat, Calciumphosphat, monobasischem und dibasischem Phosphat, Calciumsulphat, Calciumoxid, Magnesiumoxid, Magnesiumhydroxid.

7. Verfahren nach Anspruch 1,
worin die Bioadhäsive als Matrix oder Bindemittel in Prozentsätzen im Bereich von 2,0 bis 75 Gew.% vorhanden sind.

8. Verfahren nach Anspruch 1, worin der aktive Bestandteil ausgewählt wird aus der Gruppe, bestehend aus Diphenhydramin, Tripenellamin, Chlorpheniraminmaleat, Promethazin, Cimetidin, Ranitidin, Homeprasol, Prostaglandin, Carbenoxolan, Sucralphat, Chlorthiazid, Hydralazin, Isosorbid, Nitroglycerin, Metoprolol, Chlofibrat, Verapamil, Propanolol, Phenytoin, Procainamid, Amiodaron, Quinidin, Captopril, Enalapril, Nifedipin, Verapamil, Diltiazem, Nadolol, Timolol, Pindolol, Salbutamol, Terbutalin, Carbuterol, Broxaterol, Aminophyllin, Theophyllin, Cyclizin, Cinnarizin, Domperidon, Alizaprid, Vincristin, Medroxyprogesteronacetat, Megestrolacetat, Daunorubicin, Actinomycin, Adriamycin, Etoposid und Teniposid, 5-Fluorouracil, Acetylsalicylsäure, Indomethacin, Acemetacin, Sulindac, Piroxiam, Ibuprofen, Naproxen, Ketoprofen, Temazepam, Lorazepam, Flunitrazepam, Dopa, Amantadin, Protriptylin-Hydrochlorid, Trimipraminmaleat, Isocarboxazid, Nicergolin, Ampicillin, Amoxycillin, Aminoglycosiden, Cefazolam, Ceftriaxon, Erythromycin, Tetracyclinen, Acyclovir, Gancyclovir, Zidovudin, Riboflavin, Cyanokobalamin, Aminosäuren, Eisen- oder Kalciumsalzen, Salzen von essentiellen Spurenelementen.

9. Verfahren nach Anspruch 1,
worin die Zusammensetzungen in der Form von Vielkörnchenformulierungen mit einer Größe von <0,3 mm bis 3 mm hergestellt werden.

## Revendications (Revendications pour l'(les) Etat(s) contractant(s) suivant(s): AT, BE, CH, LI, DE, DK, FR, GB, IT, LU, MC, NL, PT, SE, IE)

1. Compositions pharmaceutiques solides destinées à une administration par voie orale, présentant un temps de séjour gastrique prolongé, comprenant une ou plusieurs substances inorganiques de densité élevée, une ou plusieurs substances bioadhésives, un principe actif - soit tel quel, soit mélangé avec un support -, ainsi que des excipients, liants, lubrifiants et d'autres produits classiquement utilisés dans les formulations pharmaceutiques, caractérisées en ce que lesdites substances bioadhésives sont sélectionnées dans le groupe consistant en gomme tragacanthe, gomme guar, gomme d'acacia, scléroglucane, schizophillane, xanthane, chitosan, copolymères d'anhydride maléique et de méthylvinyléther, et gel de silicone adhésif.

2. Compositions pharmaceutiques selon la revendication 1, dans lesquelles lesdites substances inorganiques présentent une densité de 2 à 7 g/cm³.

3. Compositions pharmaceutiques selon la revendication 1, dans lesquelles lesdites substances inorganiques sont utilisées sous la forme de solides particulaires présentant une dimension moyenne de 0,1 à 2,5 mm.

4. Compositions pharmaceutiques selon la revendication 1, présentant une densité de 1,5 à 4 g/cm³.

5. Compositions pharmaceutiques selon la revendication 1, dans lesquelles lesdites substances inorganiques représentent 20 à 80 % en poids.

6. Compositions pharmaceutiques selon la revendication 1, dans lesquelles lesdites substances inorganiques sont sélectionnées dans le groupe consistant en trisilicate de magnésium, oxyde de magnésium, oxyde d'aluminium, carbonate de bismuth, nitrate de bismuth, oxyde de zinc, dioxyde de titane, ferrite de calcium, oxyde ferreux, sulfate de baryum, oxyde ferrique, fer métallique, oxyde de samarium et hydroxyde de samarium, oxyde et hydroxyde d'erbium, hydroxyde d'aluminium, carbonate de calcium, silicate de calcium-aluminium, carbonate de magnésium, phosphate de calcium, mono-et di-phosphate, sulfate de calcium, oxyde de calcium, oxyde de magnésium, hydroxyde de magnésium.

7. Compositions pharmaceutiques selon la revendication 1, dans lesquelles lesdites substances inorganiques sont formulées dans la forme pharmaceutique.

8. Compositions pharmaceutiques selon la revendication 1, dans lesquelles lesdites substances inorganiques sont dispersées dans un film enrobant la ferme pharmaceutique.

9. Compositions pharmaceutiques selon la revendication 1, dans lesquelles lesdites substances bio-adhésives sont présentes en tant que matrice ou liant en des pourcentages variant de 2,0 % à 75 % en poids.

10. Compositions pharmaceutiques selon la revendication 1, dans lesquelles lesdites substances bio-adhésives sont présentes en tant que revêtement externe de la forme solide en des pourcentages variant de 1 % à 20 % en poids.

11. Compositions pharmaceutiques selon la revendication 1, dans lesquelles ledit principe actif est sélectionné dans le groupe consistant en: diphénhyldramine, tripelellamine, maléate de chlorphéniramine, prométhazine, cimétidine, ranitidine, homeprasole, prostaglandine, carbénoxolane, sucralphate, chlorothiazide, hydralazine, isosorbide, nitroglycérine, métoprolol, clofibrate, verapamil, propanol, phénytoïne, procaïnamide, amiodarone, quinidine, captopril, énalapril, nifédipine, vérapamil, diltiazem, nadolo, timolol, pindolol, salbutamol, terbutaline, carbutérol, broxatérol, aminophylline, théophylline, cyclizine, cinnarizine, dompéridone, alizapride, vincristine, acétate de médroxyprogestérone, acétate de mégestrol, daunorubicine, actinomycine, adriamycine, étoposide et téniposide, 5-fluoro-uracile, acide acétylsalicylique, indométhacine, acématacine, sulindac, piroxixam, ibuprofène, naproxène, cétoprofène, témazepam, lorazepam, flunitrazepam, dopa, amantadine, chlorhydrate de protriptyline, maléate de trimipramine, isocarboxazide, nicergoline, ampicilline, amoxycilline, aminoglycosides, céfazolam, ceftriazone, érythromycine, tétracyclines, acyclovir, gancyclovir, zidovudine, riboflavine, cyanocobalamine, acides aminés, sels de fer ou de calcium, sels d'oligo-éléments essentiels.

12. Compositions pharmaceutiques selon la revendication 1, préparées sous la forme de comprimés.

13. Compositions pharmaceutiques selon la revendication 1, préparées sous la forme de formulations multiparticulaires présentant une dimension allant d'une valeur inférieure à 0,3 mm à une valeur de 3 mm.

14. Compositions pharmaceutiques selon la revendication 1, comprenant un noyau exempt de substance bio-adhésive revêtu d'un film de substance bio-adhésive.

## Revendications (Revendications pour l'(les) Etat(s) contractant(s) suivant(s): ES, GR)

1. Procédé de préparation de compositions pharmaceutiques solides destinées à une administration par voie orale, présentant un temps de séjour gastrique prolongé, comprenant une ou plusieurs substances inorganiques de densité élevée, une ou plusieurs substances bio-adhésives, un principe actif - soit tel quel, soit mélangé avec un support -, ainsi que des excipients, liants, lubrifiants et d'autres produits classiquement utilisés dans les formulations pharmaceutiques, dans lequel lesdits ingrédients à densité élevée sont mélangés à sec, par agitation, avec lesdits principes actifs, les substances bio-adhésives, les liants, les excipients, les lubrifiants et autres produits, et le mélange est converti en compositions sous formes unitaires individuelles, comprimés ou en formulations multiparticulaires, pilules, granulés, microgranulés et microparticules.

2. Procédé selon la revendication 1, dans lequel lesdites substances bio-adhésives sont sélectionnées dans le groupe consistant en: gomme tragacanthe, gomme guar, gomme d'acacia, scléroglucane, schizophillane, xanthane, chitosan, copolymères d'anhydride maléique et de méthylvinyléther, et gel de silicone adhésif.

3. Procédé selon la revendication 1, dans lequel lesdites substances inorganiques présentent une densité de 2 à 7 g/cm³.

4. Procédé selon la revendication 1, dans lequel lesdites substances inorganiques sont utilisées sous la forme de solides particulaires présentant un diamètre moyen de 0,1 à 2,5 mm.

5. Procédé selon la revendication 1, dans lequel lesdites substances inorganiques représentent de 20 % à 80 % en poids.

6. Procédé selon la revendication 1, dans lequel lesdites substances inorganiques sont sélectionnées dans le groupe consistant en trisilicate de magnésium, oxyde de magnésium, oxyde d'aluminium, carbonate de bismuth, nitrate de bismuth, oxyde de zinc, dioxyde de titane, ferrite de calcium, oxyde ferreux, sulfate de baryum, oxyde ferrique, fer métallique, oxyde de samarium et hydroxyde de samarium, oxyde et hydroxyde d'erbium, hydroxyde d'aluminium, carbonate de calcium, silicate de calcium-aluminium, carbonate de magnésium, phosphate de calcium, mono- et diphosphates, sulfate de calcium, oxyde de calcium, oxyde de magnésium, hydroxyde de magnésium.

7. Procédé selon la revendication 1, dans lequel lesdites substances bio-adhésives sont présentes en tant que matrice ou liant en des pourcentages variant de 2,0 % à 75 % en poids.

8. Procédé selon la revendication 1, dans lequel ledit principe actif est sélectionné dans le groupe consistant en: diphénhyldramine, tripelellamine, maléate de chlorphéniramine, prométhazine, cimétidine, ranitidine, homeprasole, prostaglandine, carbénoxolane, sucralphate, chlorothiazide, hydralazine, isosorbide, nitroglycérine, métoprolol, clofibrate, verapamil, propanol, phénytoïne, procaïnamide, amiodarone, quinidine, captopril, énalapril, nifédipine, vérapamil, diltiazem, nadolo, timolol, pindolol, salbutamol, terbutaline, carbutérol, broxatérol, aminophylline, théophylline, cyclizine, cinnarizine, dompéridone, alizapride, vincristine, acétate de médroxyprogestérone, acétate de mégestrol, daunorubicine, actinomycine, adriamycine, étoposide et téniposide, 5-fluoro-uracile, acide acétylsalicylique, indométhacine, acématacine, sulindac, piroxixam, ibuprofène, naproxène, cétoprofène, témazepam, lorazepam, flunitrazepam, dopa, amantadine, chlorhydrate de protriptyline, maléate de trimipramine, isocarboxazide, nicergoline, ampicilline, amoxycilline, aminoglycosides, céfazolam, ceftriazone, érythromycine, tétracyclines, acyclovir, gancyclovir, zidovudine, riboflavine, cyanocobalamine, acides aminés, sels de fer ou de calcium, sels d'oligo-éléments essentiels.

9. Procédé selon la revendication 1, dans lequel lesdites compositions sont préparées sous la forme de formulations multi-particulaires présentant une dimension allant d'une valeur inférieure à 0,3 mm à une valeur de 3 mm.
